# EUROPÄISCHE PATENTSCHRIFT

(11) **EP 1 247 091 B1**
(45) Veröffentlichungstag und Bekanntmachung des Hinweises auf die Patenterteilung: **29.10.2008**
(21) Anmeldenummer: 00991287.4
(22) Anmeldetag: 21.12.2000
(51) Int. Cl.: G01N 27/90, G01N 29/26, G01B 7/12

(54) **PRÜFEINRICHTUNG ZUM PRÜFEN VON LANGGESTRECKTEN GEGENSTÄNDEN**
TEST DEVICE FOR TESTING LONG OBJECTS
DISPOSITIF DE CONTROLE SERVANT A CONTROLER DES OBJETS ALLONGES

(30) Priorität: 27.12.1999 DE 19963231
(43) Veröffentlichungstag der Anmeldung: 09.10.2002
(73) Patentinhaber: Institut Dr. Friedrich Förster Prüfgerätebau GmbH & Co. KG, 72766 Reutlingen (DE)
(72) Erfinder: HÄBERLEIN, Peter, 72768 Reutlingen (DE)
(74) Vertreter: Patentanwälte Ruff, Wilhelm, Beier, Dauster & Partner
(86) Internationale Anmeldenummer: PCT/EP2000/013390
(87) Internationale Veröffentlichungsnummer: WO 2001/048468

(56) Entgegenhaltungen:
- DE-A- 2 847 716
- DE-A- 3 739 190
- DE-A- 4 314 274
- DE-A- 4 324 332
- DE-U- 9 108 162
- GB-A- 2 034 049
- US-A- 3 582 771

## Beschreibung

Die Erfindung betrifft eine Prüfeinrichtung zum Prüfen von langgestreckten Gegenständen gemäß dem Oberbegriff von Anspruch 1.

Derartige Prüfeinrichtungen werden in der zerstörungsfreien Prüftechnik insbesondere zur Prüfung von langgestrecktem Halbzeug, wie Drähten, Stangen, Rohren o. dgl. auf Fehler eingesetzt. Dabei kommen vorwiegend Wirbelstrom- oder Streufluß-Prüfsonden zur Anwendung, es sind jedoch auch andere, ggf. nicht magnetisch arbeitende Prüfsonden nutzbar, die beispielsweise mit Ultraschall arbeiten.

Eine Prüfeinrichtung dieser Art hat einen zum Hindurchführen des zu prüfenden Gegenstandes ausgebildeten, um eine Rotierkopfachse drehbaren Rotierkopf, an dem mindestens eine an beweglichen Sonderhaltemitteln angeordnete, auf kreisförmigen Umlaufbahnen um den Gegenstand führbare Prüfsonde zur zerstörungsfreien Prüfung oberflächennaher Bereiche des Gegenstandes vorgesehen ist. In der Regel sind mehrere, beispielsweise vier Prüfsonden und eine entsprechende Anzahl von Sondehaltemitteln vorgesehen.

Es können zwei Arbeitsweisen derartiger Prüfeinrichtungen unterschieden werden, nämlich eine berührungslose und eine schleifende Prüfung. Bei der berührungslosen Prüfung wird zwischen Prüfsonde und Oberfläche des Prüfgegenstandes ein möglichst konstanter, in der Regel sehr geringer Abstand, beispielsweise in der Größenordnung von ca. 1 mm eingehalten, wozu die Sondenhaltemittel in eine entsprechende Stellung gebracht werden. Insbesondere dort, wo Abstandsschwankungen, die sich zwangsläufig aus Unrundheit, Exzentrizität oder Durchmessertoleranzen des Prüfteiles ergeben, nicht akzeptabel sind und/oder wo es beispielsweise aufgrund des Meßprinzips auf minimalen Abstand und/oder Berührungskontakt zum Prüfgegenstand ankommt, wird mit schleifender Prüfung gearbeitet. Bei dieser steht eine dazu besonders befähigte Gleitfläche, hinter der sich die Prüfsonde befindet, in unmittelbarem Berührungskontakt mit der Oberfläche des Prüfteiles und macht, unter Bewegung der beweglichen Sondenhaltemittel, alle Auslenkungen dieser Oberfläche mit.

Bei gattungsgemäßen Prüfeinrichtungen ist eine Verstelleinrichtung zum Verstellen des Prüfdurchmessers, also des Durchmessers der Prüfsondenumlaufbahn vorgesehen, mit deren Hilfe bei unterschiedlichen Durchmessern von Prüfteilen eine optimale Prüfstellung der Prüfsonde erreicht werden kann. Da in der Regel die Meßempfindlichkeit der Prüfeinrichtung sehr empfindlich vom Abstand zwischen Prüfsonde und Oberfläche des Gegenstandes abhängt, ist eine möglichst exakte Durchmessereinstellung erforderlich, um beispielsweise bei berührungsloser Messung einen geeigneten Radialabstand zwischen Prüfsonde und Gegenstandsoberfläche einzustellen oder um bei berührender Messung die Sondenhaltemittel so einzustellen, daß der Berührungsbereich zwischen Gleitfläche und Oberfläche möglichst genau am Ort der Prüfsonde liegt. Prüfeinrichtungen mit derartigen Verstelleinrichtungen sind beispielsweise in der DE 37 39 190 oder der DE 43 24 332 gezeigt.

Bei diesen herkömmlichen Prüfeinrichtungen ist die Durchmesserverstellung relativ arbeitsaufwendig und erfordert einen Eingriff in das Innere der üblicherweise mit einem Gehäuse nach außen abgeschlossenen Prüfeinrichtung. So ist beispielsweise bei der Prüfeinrichtung der DE 37 39 190 am Umfang des Rotierkopfes eine mittels eines Imbusschlüssels betätigbare Stellschraube vorgesehen, die über eine Kardanwelle auf gegeneinander bewegliche Elemente der Verstelleinrichtung wirkt. Zur Verstellung wird nach Anhalten des Rotierkopfes das Gehäuse geöffnet und es werden zunächst Feststellschrauben gelöst, die die gegeneinander beweglichen Elemente der Verstelleinrichtung nach erfolgter Durchmesserverstellung in der gewünschten Relativstellung festsetzen. Vor oder nach diesem Arbeitsschritt wird der Rotor manuell so gedreht, daß die Stellschraube zugänglich und eine im Bereich der Stellschraube angeordnete Skala zur Überprüfung des eingestellten Durchmessers sichtbar ist. Anschließend erfolgt mittels eines Imbusschlüssels durch Drehung der Stellschraube die Durchmesserverstellung, bei der sich die freigesetzten Elemente der Verstelleinrichtung gegeneinander bewegen. Nach erfolgter Durchmessereinstellung werden die Feststellschrauben wieder angezogen, das Gehäuse wird geschlossen und ein neuer Messvorgang mit verändertem Prüfdurchmesser kann stattfinden.

Bei der Prüfeinrichtung der DE 43 14 274 hat die Verstelleinrichtung ein von außerhalb der Prüfeinrichtung zugängliches Betätigungsorgan in Form eines Kupplungselementes, an das ein externer Verstellantrieb mit Hilfe einer Mehrkantwelle angekoppelt werden kann.

Der Erfindung liegt die Aufgabe zugrunde, eine Prüfeinrichtung der eingangs erwähnten Art zu schaffen, die bei einfacher Handhabung eine sichere, schnelle und präzise Durchmessereinstellung ermöglicht.

Diese Aufgabe wird gelöst durch eine Prüfeinrichtung mit den Merkmalen von Anspruch 1. Bevorzugte Weiterbildungen sind in den abhängigen Ansprüchen angegeben. Der Wortlaut sämtlicher Ansprüche wird durch Bezugnahme zum Inhalt der Beschreibung gemacht.

Bei einer erfindungsgemäßen Prüfeinrichtung hat die Verstelleinrichtung mindestens ein von außerhalb der zugängliches Betätigungsorgan zur verstellwirksamen Betätigung der Verstelleinrichtung. Durch die Zugänglichkeit von außen ist es möglich, die Verstelleinrichtung ohne die herkömmlich erforderliche Öffnung eines Gehäuses der Prüfeinrichtung vorzunehmen. Das vorzugsweise permanent, d. h. auch bei Betrieb der Einrichtung, zugängliche Betätigungsorgan hat also betätigungsrelevante Abschnitte, die sich in einem ungefährlichen Bereich außerhalb rotierender bzw. sich schnell bewegender Teile der Prüfeinrichtung befinden. Dadurch ist neben einer manuellen Betätigung der Verstelleinrichtung insbesondere auch eine Automatisierung des Verstellvorganges möglich, indem beispielsweise ein geeigneter automatischer Manipulator vorgesehen wird, der an dem Betätigungsorgan angreift und die zur Verstellung erforderlichen Betätigungen des Betätigungsorganes durchführt. Eine derartige Manipulationseinrichtung kann einfach und kostengünstig aufgebaut sein, insbesondere weil zur Verstellung keine Eingriffe in das Gehäuse der Prüfeinrichtung erforderlich sind.

Weiterhin ist die Verstelleinrichtung, insbesondere durch Bewegung des Betätigungsorgans, zwischen mehreren Verstellzuständen umschaltbar, wobei in einem ersten Verstellzustand, der auch als Ruhezustand bezeichnet wird, kein Verstelleingriff mit dem Rotierkopf vorliegt, in einem zweiten Verstellzustand durch Bewegung des Betätigungsorgans eine kontrollierte Rotation des Rotierkopfs durchführbar ist, und in einem dritten Verstellzustand eine Durchmesserverstellung durch führbar ist.

Bei einer bevorzugten Ausführungsform reicht ein einziges Betätigungsorgan zur Durchmesserverstellung und zur Umschaltung zwischen verschiedenen Verstellzuständen aus. An diesem Multifunktionsorgan kann manuell oder maschinell angegriffen werden. Zweckmäßig hat das Betätigungsorgan mehrere Verstellfreiheitsgrade, indem es einerseits um eine Organachse drehbar und, unabhängig von der Drehung, parallel zur Organachse verschiebbar gelagert ist. In diesem Fall reichen sehr einfach sowohl manuell, als auch maschinell realisierbare Stellbewegungen aus, um sämtliche Funktionen der Verstelleinrichtung durchzuführen.

Eine bevorzugte Weiterbildung zeichnet sich dadurch aus, daß die Verstelleinrichtung als vollmechanische Verstelleinrichtung ausgebildet ist. Die Verstelleinrichtung kann also vollständig frei von elektrisch ansteuerbaren Stellgliedern o. dgl. aufgebaut sein, die insbesondere in rauhen Arbeitsumgebungen störungsanfällig sein können. Eine vollmechanische Verstelleinrichtung kann demgegenüber nicht nur sehr robust, sondern auch besonders kostengünstig herstellbar sein. Dabei ist das Betätigungsorgan beweglich gelagert und derart mit verstellwirksam beweglichen Elementen der Verstelleinrichtung mechanisch gekoppelt oder koppelbar, daß durch Bewegung des Betätigungsorgans eine Verstellung des Prüfdurchmessers, und auch eine Umschaltung der Verstelleinrichtung zwischen verschiedenen Verstellzuständen durchführbar ist. Es ist auch möglich, mindestens ein Betätigungsorgan als von außen zugänglichen Schalter, Drehknopf, Schieber o. dgl. auszubilden, mit dessen Hilfe fremdenenergiebetätigte Stellorgane angesteuert werden, die z. B. elektrisch, elektromagnetisch, pneumatisch oder hydraulisch arbeiten.

Zur Erhöhung der Bedien- und Betriebssicherheit ist bei einer Weiterbildung vorgesehen, daß der Verstelleinrichtung eine Sicherungseinrichtung zugeordnet ist, die derart ausgelegt ist, daß eine Bewegung des Betätigungsorgans in einen Verstellzustand mit verstellwirksamem Eingriff zwischen Betätigungsorgan und Rotierkopf nur bei im wesentlichen unbewegten, d. h. nicht drehenden Rotierkopf durchführbar ist. Damit wird zuverlässig eine ungewollte Beschädigung der Prüfeinrichtung und eine ggf. damit einhergehende Verletzung eines Bedieners vermieden. Dies ist besonders bei modernen Prüfmaschinen vorteilhaft, die für eine schnelle und möglichst lückenlose Prüfung der Gegenstände mit typischen Drehzahlen des Rotierkopfes von mehreren tausend Umdrehungen pro Minute, beispielsweise bis in die Größenordnung von ca. 9.000 bis ca. 9.500 Umdrehungen pro Minute arbeiten.

Obwohl eine derartige Sicherungseinrichtung zumindest teilweise auch mit elektrisch betreibbaren Komponenten aufgebaut sein kann, ist sie bei einer bevorzugten Weiterbildung vollmechanisch, so daß sie beispielsweise auch bei Ausfall einer elektrischen Energieversorgung arbeitsfähig bleibt. Eine besonders wirkungsvoll sowie weitgehend verschleißfrei arbeitende Sicherungseinrichtung mit einem nach Art einer Sperrklinke ausgebildeten Sperrorgan wird im Zusammenhang mit dem Ausführungsbeispiel näher erläutert. Das Sperrorgan ist, insbesondere durch anschlagsbegrenzte Bewegung in Radialrichtung, in Wirkverbindung mit dem Rotierkopf bewegbar und durch Drehung des Rotierkopfes in eine das Betätigungsorgan sperrende Sperrstellung bewegbar, insbesondere verschwenkbar. Dabei kann das Sperrorgan derart leichtgängig gelagert sein, daß es bei Annäherung an den Rotierkopf und ausreichend hoher Drehzahl des Rotierkopfes allein durch Luftzug in die Sperrstellung bewegbar ist, so daß ein Berührungskontakt zum Rotierkopf bei hohen Drehzahlen nicht stattfindet. Durch das Sperrorgan wird auf vollmechanischem Wege verhindert, daß das Betätigungsorgan in Eingriff mit drehenden Teilen des Rotierkopfes gebracht wird.

Als weitere Sicherung kann eine Einrichtung vorgesehen sein, mit deren Hilfe die elektrische Versorgung eines elektrischen Prüfkopfantriebs automatisch unterbrochen wird, wenn das Betätigungsorgan aus seiner Ruhestellung in einen Verstellzustand mit verstellwirksamem Eingriff zum Rotierkopf bewegt wird. Hierzu kann ein durch das Betätigungsorgan schaltbarer, elektrischer Sicherheitsschalter vorgesehen sein. Dadurch kann ein Anlaufen des Rotorantriebs verhindert werden, selbst wenn noch kein formschlüssiger Eingriff zwischen Betätigungsorgan und Rotierkopf vorliegt.

Zur Erzielung aussagekräftiger Meßergebnisse ist eine exakte Einstellung des Prüfdurchmessers erforderlich. Dies wird bei einer bevorzugten Weiterbildung dadurch gefördert, daß die Verstelleinrichtung spielfrei einstellbar ist. Zweckmäßig wird dieses durch eine geeignete Selbsthemmung von gegeneinander beweglichen Elementen der Verstelleinrichtung erreicht.

Bei einer bevorzugten Weiterbildung hat der Rotierkopf einen um die Rotierkopfachse drehbaren Rotor zum Tragen der Sondenhaltemittel und mindestens ein koaxial mit dem Rotor angeordnetes, relativ zum Rotor um die Prüfkopfachse verdrehbares, vorzugsweise nach Art einer Kurvenscheibe ausgebildetes Verstellelement, das derart mit den Sondenhaltemitteln gekoppelt ist, daß die Sondenhaltemittel durch Relativverdrehung zwischen Rotor und Verstellelement zur Änderung des Prüfdurchmessers verstellbar sind. Zur Realisierung der Selbsthemmung sind der Rotor und das Verstellelement mittels einer Reibschlußverbindung reibschlüssig miteinander verbunden. Durch den Reibschlußkontakt zwischen Rotor und Verstellelement wirkt sich ein ggf. vorhandenes Spiel im Kraftübertragungsstrang zwischen Betätigungsorgan und der Rotor/Verstellelement-Kombination nicht auf das Einstellspiel aus. Eine Verstellung ist nur dann möglich, wenn zwischen Rotor und am Verstellelement Drehmomente angreifen, die zur Überwindung der Reibungskraft im Bereich der Reibschlußverbindung ausreichen. Eine einmal eingestellte Relativstellung bleibt ohne weitere Maßnahmen zuverlässig gesichert. Der gegenseitige Angriff zwischen Rotor und Verstellelement kann ausschließlich reibschlüssig bzw. kraftschlüssig sein, so daß eine herkömmlich vorgesehene Feststellung der gegeneinander beweglichen Elemente mittels Feststellschrauben o. dgl. entfallen kann.

Vorteilhaft ist die Reibschlußverbindung derart ausgelegt, daß eine Reibungskraft erzeugbar ist, die mit zunehmender Drehzahl des Rotierkopfes zunimmt. Dadurch kann erreicht werden, daß im Betrieb, d.h. bei hohen Drehzahlen, der Rotor und das Verstellelement praktisch unverrückbar aneinander angreifen, während bei Stillstand des Rotors, der Voraussetzung für eine Durchmesserverstellung ist, die Reibungskraft so gering wird, daß sie ohne große Mühe zur Durchmesserverstellung überwindbar ist. Eine bevorzugte Ausführungsform mit einer derartigen Selbsthemmung der Verstelleinrichtung und drehzahlabhängiger Kraftschlußverbindung zwischen Rotor und Verstellelement, bei der zwischen Rotor und Verstellelement mindestens ein gesondertes, elastisches Reibschlußelement z.B. nach Art eines Dichtringes vorgesehen ist, wird anhand des Ausführungsbeispiels näher beschrieben. Die hier beschriebene Kombination aus Rotor und Verstellelement, die ggf. über einen geschwindigkeitsabhängigen Reibschluß miteinander gekoppelt sind, kann bei allen gattungsgemäßen Prüfeinrichtungen vorteilhaft sein, unabhängig von der Art des Betätigungsorgans.

Ein weiterer Aspekt der Erfindung betrifft die Funktionsfähigkeit der Verstelleinrichtung, die bei herkömmlichen Einrichtungen insbesondere durch Verschmutzung von kritischen Bereichen der Verstelleinrichtung im Betrieb beeinträchtigt wird. Bei bevorzugten Weiterbildungen ist die Verstelleinrichtung im wesentlichen vollständig verschmutzungsgeschützt abgedichtet. Dies bedeutet insbesondere, daß durch geeignete Abdichtmittel im wesentlichen alle gegeneinander beweglichen Elemente der Verstelleinrichtung, und/oder die Berührungsbereiche zwischen aneinander angreifenden Elementen der Verstelleinrichtung, verschmutzungsgeschützt sind. Hiervon können ggf. solche Elemente ausgenommen sein, die unmittelbar an den Sondehaltemitteln angreifen und hierzu mindestens teilweise in den Prüfsondenraum der Prüfeinrichtung hineinragen, also in denjenigen Raum, in dem die Prüfsonden umlaufen. In diesem nahe am Eingang der Prüfeinrichtung liegenden Prüfsondenraum können beisielsweise Zunderreste oder andere vom einlaufenden Gegenstand mitgeführte Verschmutzungspartikel umherwirbeln. Durch die Abdichtmittel wird ein Eindringen derartiger Verschmutzungspartikel in das Innere der Prüfeinrichtung, insbesondere auch in das Innere der Verstelleinrichtung, zuverlässig vermieden. Bevorzugte Abdichtmittel werden im Zusammenhang mit dem Ausführungsbeispiel näher beschrieben. Sie umfassen insbesondere eine Labyrinthdichtung am Umfang des Rotierkopfes zwischen Rotierkopf und Gehäuse sowie abgedichtet durch einen Rotorabschnitt geführte Verstellglieder für die Sondenhaltemittel, wobei die Verstellglieder an der Vorderseite des Rohrabschnitts im Prüfsondenraum an den Sondenhaltemitteln angreifen und im Bereich der Rückseite des Rotorabschnittes in das dort angeordnete Verstellelement (Kurvenscheibe) eingreifen. Die erläuterten Merkmale und Merkmalskombinationen, durch die ein Schutz der Verstelleinrichtungen gegen Verschmutzung sichergestellt wird, können bei allen gattungsgemäßen Prüfeinrichtungen unabhängig von der Art des Betätigungsorgans vorteilhaft sein.

Die Erfindung ist bei verschiedenen unterschiedlichen Typen von Prüfeinrichtungen mit Vorteil einsetzbar. Insbesondere können die die Prüfsonden tragenden Sondenhaltemittel direkt mit einer Kurvenscheibe oder einem anderen gleich wirkenden Verstellelement in Eingriff stehen, wie es beispielsweise bei Rotierköpfen mit radial verstellbaren Stiftsonden der Fall ist. Die Erfindung ist auch bei Rotierköpfen mit Hebel-Sondenanordnungen nutzbar, also solchen Einrichtungen, bei denen das Verstellelement auf ein Hebelsystem wirkt, das unter anderem als Sondenhaltemittel sogenannte Sondenhaltehebel umfaßt, die um eine parallel zur Rotationsachse verlaufende Schwenkachse schwenkbar sind.

Bei einer bevorzugten Ausführungsform ist vorgesehen, daß die Sondenhaltemittel, insbesondere die Schwenkhebel, abhängig von der Drehzahl des Rotierkopfes selbsttätig zwischen einer gegenstandsnahen Stellung und einer Abhebestellung unabhängig von der Einstellung der Verstelleinrichtung derart verstellbar sind, daß unterhalb einer Grenzdrehzahl die Abhebestellung eingenommen wird. Dies wird bevorzugt dadurch erreicht, daß ein Sondenhebel, insbesondere mittels einer Blattfeder, in die Abhebestellung vorgespannt ist und daß ein Sondenhaltehebel derart außerhalb seines Massenschwerpunktes gelagert ist, das abhängig von auftretenden Fliehkräften ein in Richtung der gegenstandsnahen Stellung wirkendes Drehmoment auftritt. Dies ermöglicht eine selbsttätige, drehzahlabhängige Zustellung bzw. Abhebung der Prüfsonden, was anhand des Ausführungsbeispiels näher erläutert wird.

Eine besonders im Zusammenhang mit derartigen Ausführungsformen vorteilhafte Ausführungsform der Verstelleinrichtung weist für jedes Sondenhaltemittel ein diesem zugeordnetes Verstellglied auf, das als verstellbarer Anschlag zur Festlegung der Position der gegenstandsnahen Stellung des Sondenhaltemittels bzw. der Prüfposition ausgebildet ist. Durch diesen Anschlag wird die Annäherung an den Gegenstand zuverlässig begrenzt und durch Verstellung des Anschlages ist der Prüdurchmesser verstellbar. Die Möglichkeit der (fliehkraftunterstützten) Selbstabhebung bleibt erhalten. Bevorzugt ist der Anschlag als Innenanschlag an einer gegenstandsnahen Position des Sondenhaltemittels angeordnet, greift also in unmittelbarer Nähe der Prüfsonden am Sondenhaltemittel an. Dies hat insbesondere den Vorteil, daß sich der eingestellte Durchmesser bzw. die eingestellte Sondenposition praktisch nicht ändert, auch wenn insbesondere bei hohen Drehzahlen fliehkraftbedingt und bedingt durch Luftbewegungen Kräfte auf die Sondenhaltemittel einwirken, die zu einer Verbiegung der Sondenhaltemittel führen können. Somit tritt praktisch keine Drift der Prüfempfindlichkeit auf. Außerdem wirken sich aufgrund des langen Hebelarmes zur Schwenkachse des Sondenhaltemittels Toleranzen bei der Einstellung des Anschlages nur geringfügig auf den eingestellten Durchmesser aus. Verstellbare Anschläge dieser Art können bei allen gattungsgemäßen Prüfeinrichtungen unabhängig von der Art der Betätigung der Verstelleinrichtung vorteilhaft sein.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte Ausführungsformen darstellen können. Dies gilt insbesondere für diejenigen Merkmale und Merkmalskombinationen, die die verschmutzungsdichte Abdichtung zwischen Prüfsondenraum und Gehäuseinnenraum sowie die Selbsthemmung zwischen Rotor und Verstellelement betreffen.

Ein Ausführungsbeispiel der Erfindung wird in den Zeichnungen dargestellt und im folgenden näher erläutert. Es zeigen:
- Fig. 1: eine Seitenansicht in teilweisem Längsschnitt durch eine Ausführungsform einer erfindungsgemäßen Prüfeinrichtung;
- Fig. 2: eine axiale Ansicht der Prüfeinrichtung in Fig. 1 in Durchlaufrichtung zu prüfender Gegenstände;
- Fig. 3: einen horizontalen Schnitt durch einen Teil einer bevorzugten Ausführungsform einer Durchmesser-Verstelleinrichtung mit einem von außerhalb der Prüfeinrichtung zugänglichen Betätigungsorgan mit Handrad und
- Fig. 4: eine Axialansicht mit einem vertikalen Schnitt durch die in Fig. 3 gezeigte Einrichtung zur Erläuterung der Funtion einer vollmechanischen Sicherungseinrichtung für die Verstelleinrichtung.

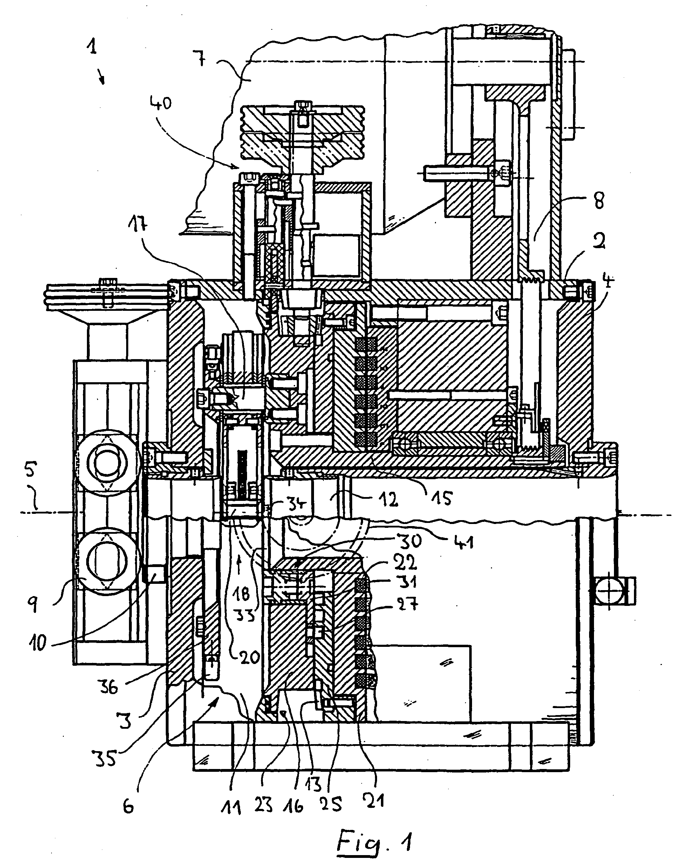

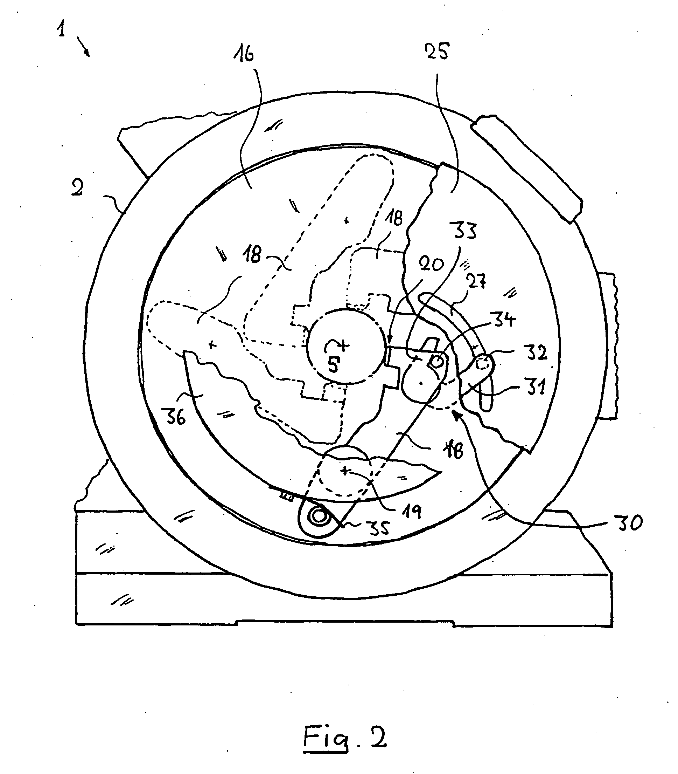

Die Seitenansicht in Fig. 1 zeigt eine bevorzugte Ausführungsform einer erfindungsgemäßen Wirbelstrom-Prüfeinrichtung 1 im teilweisen vertikalen Längsschnitt. Die Prüfeinrichtung hat ein im wesentlichen zylindrische Stahlgehäuse 2, das an der Vorderseite und der Rückseite durch in ihrer Mitte durchbrochenen Platten 3 bzw. 4 geschlossen ist. Ein nicht gezeigter Prüfgegenstand, beispielsweise eine zylindrische Stange, durchläuft im Prüfbetrieb die Prüfeinrichtung von der Eintrittsseite bei Vorderplatte 3 zur Austrittsseite bei Rückplatte 4 koaxial zur Rotationsachse 5 eines innerhalb des Gehäuses 2 angeordneten, mehrteiligen Rotierkopfes 6, der mittels eines oberhalb des Gehäuses 2 angeordneten Elektromotors 7 über eine Kraftübertragung 8 drehend antreibbar ist. Der Prüfgegenstand wird beim Durchlauf eingangsseitig durch Zentriermittel in Form von Rollen 9 und eine nachgeschaltete, in die Frontabdeckung 3 eingesetzte Führungsdüse 10 geführt, durchläuft danach den eigentlichen Prüfbereich oder Prüfraum 11, in dem vier im Zusammenhang mit Fig. 2 näher erläuterte Prüfsonden auf kreisförmigen Umlaufbahnen um den Gegenstand herumgeführt werden und verläßt die Prüfeinrichtung unter Führung durch eine dem Prüfraum 11 nachgeschaltete Führungsdüse 12, die in einem zylindrischen Kanal im Inneren des Rotierkopfes sitzt.

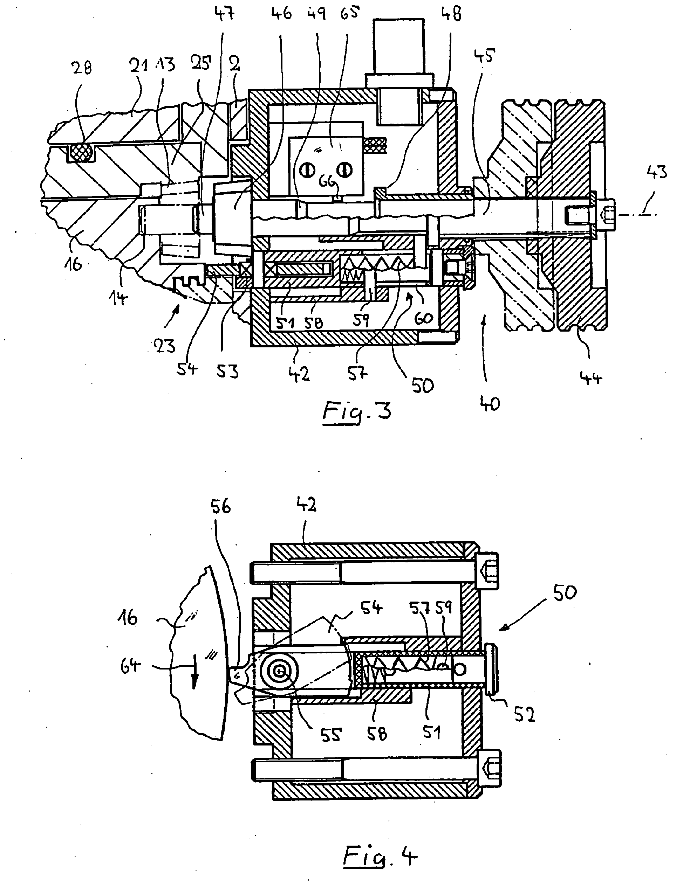

Der mehrteilig aufgebaute Rotierkopf bzw. Prüfkopf hat als tragendes Element eine auch als Rotor bezeichnete Hohlwelle 15, die eingangsseitig einen scheibenförmigen Rotorabschnitt 16 aufweist, dessen Umfang bis in die Nähe der Gehäuseinnenseite reicht. Am Umfang des Rotorabschnittes 16 ist eine radiale Sacklochbohrung 14 vorgesehen, deren Funktion im Zusammenhang mit Fig. 4 näher erläutert wird. In der Nähe des Umfangsrandes des Rotorabschnitts 16 ist eine umlaufende Labyrinthdichtung 23 vorgesehen, die ein Eindringen von Verschmutzungspartikeln aus dem Prüfsondenraum 11 in den hinter dem Rotorabschnitt 16 liegenden Bereich zuverlässig verhindert. Die Labyrinthdichtung umfaßt einen gehäusefest montierten Ring, der auf seiner dem Rotorabschnitt 16 zugewandten Seite mehrere koaxiale Ringnuten aufweist, die von umlaufenden Bunden begrenzt werden. Am Umfang des Rotorabschnitts 16 ist auf dessen Vorderseite eine komplementäre Anordnung von Ringnuten und Ringbunden derart angeordnet, daß die Nuten und Bünde unter Bildung eines im Schnitt mäanderförmigen Spaltes von ca. 0,5 mm Breite ineinandergreifen. Verschmutzungspartikel können durch diesen komplex geformten Durchgang insbesondere bei höheren Drehzahlen praktisch nicht vom Prüfkopfraum 11 in das Innere des Gehäuses 2 gelangen, das dadurch in diesem Bereich mittels der Labyrinthdichtung verschmutzungsdicht abgedichtet ist.

An der Einlaufseite oder Vorderseite des Rotorabschnitts sind vier gleichmäßig umfangsversetzte, koaxial zur Rotorachse 5 ausgerichtete Lagerbolzen 17 angeschraubt, an denen jeweils ein Schwenkhebel 18 um eine achsparallel zur Achse 5 verlaufende Schwenkachse 19 verschwenkbar gelagert sind. Die als Sondenhaltemittel dienenden zweiarmigen Schwenkhebel 18 sind in Leichtbauweise mit verwindungssteifen Elementen aus Kohlenstoff-Faserwerkstoff aufgebaut und tragen an ihrem näher an der Durchlaufachse 5 angeordneten Ende jeweils eine mittels einer Keramikplatte gepanzerte Prüfsonde 20. Die Prüfsonden sind im Beispiel als Wirbelstromsonden ausgebildet, können bei anderen Ausführungsformen aber auch nach anderen magnetischen oder nicht magnetischen Prinzipien arbeiten und aus einem oder mehreren Sondenelementen aufgebaut sein. Die Prüfsonden stehen über nicht gezeigte Kabel und Steckkontakte in signalleitender Verbindung mit Rotierübertragerelementen 22, die an einem drehfest mit dem Rotor montierten, scheibenförmigen Träger 21 angeordnet sind und mit entsprechenden, gehäusefesten Rotierübertrageelementen 22 eine berührungslose Signalübertragung zu nicht gezeigten Signalverarbeitungs- und Auswerteeinrichtungen sicherstellen.

Zwischen dem scheibenförmigen Rotorabschnitt 16 und dem drehfest mit diesem verbundenen Träger 21 ist ein scheibenförmiges Verstellelement 25 angeordnet, das relativ zum Rotor 15 um die Achse 5 verdrehbar ist und an seinem Umfang einen dem Rotorabschnitt 16 zugewandten Schrägzahnkranz 13 aufweist. Das nach Art einer Kurvenscheibe arbeitende Verstellelement 25 ist ein wesentliches Element einer Verstelleinrichtung zur Einstellung des Prüfdurchmessers der Prüfeinrichtung, d.h. zur Einstellung der radialen Position der Prüfsonden 19 in Bezug auf die Rotorachse 5. Wie in Fig. 2 schematisch gezeigt ist hat das Verstellelement 25 auf seiner dem Rotorabschnitt 16 zugewandten Vorderseite für jede Prüfsonde eine Spiralsegmentnut 27, deren Radialabstand zur Drehachse 5 sich in Umfangsrichtung ändert. Die Spiralscheibe 25 ist über ein Verstellglied 30 in Form eines zweiarmigen Schwenkhebels mechanisch mit einem zugeordneten Schwenkhebel 18 gekoppelt.

In Zusammenschau der Figuren 1 und 2 ist zu erkennen, daß der im Rotorabschnitt 16 um eine parallel zur Achse 5 verlaufende Schwenkachse drehbar gelagerte Schwenkhebel 30 auf der dem Verstellelement 25 zugewandten Rückseite einen in einer Ausnehmung beweglichen hinteren Hebelarm 31 hat, der über einen Koppelbolzen 32 in die zugehörige Spiralnut 27 im wesentlichen spielfrei eingreift. Der hintere Hebelarm 31 ist über eine Art Klauenkupplung drehfest mit einem vorderen Hebelarm 33 verbunden, der an der die Prüfsonden tragenden Vorderseite des Rotorabschnitts 16 angeordnet ist und über einen am Sondenhaltehebel vorgesehenen Bolzen 34 am Sondenhaltehebel angreift. Dabei ist der vordere Hebelarm 33 generell zwischen Bolzen 34 und Prüfkopfachse 5 angeordnet und dient als Innenanschlag zur Begrenzung der Bewegung des Sondenhebels bzw. der Prüfsonden in Richtung Prüfgegenstand. Das Verstellglied 30 ist mittels eines Lagerringes in der zylindrischen Durchgangsbohrung des Rotorabschnitts 16 derart abgedichtet gelagert, daß im Bereich der Verstellglieder 30 praktisch keine Verschmutzungsteilchen aus dem Prüfsondenraum 11 in das Innere des Gehäuses, insbesondere in den Bereich zwischen Rotorabschnitt 16 und Verstellelement 25, gelangen können.

Die Sondenhaltehebel 18 werden im Ruhezustand des Rotierkopfes mit Hilfe von Blattfedern 35 in Richtung auf Abstandsvergrößerung zwischen Durchlaufachse 5 und Prüfsonde 19 vorgespannt. Die Blattfedern sitzen am Rand einer in Fig. 2 ausschnittsweise und in Fig. 1 im Schnitt gezeigten Federträgerscheibe 36, die drehfest mit dem Rotor 15 verbunden und auf der dem Rotorabschnitt 16 abgewandten Seite der Sondenhaltehebel zwischen der Frontplatte 3 des Gehäuses und den Sondenhaltehebeln angeordnet ist.

Die Sondenhaltehebel sind mittels Ausgleichgewichten so ausgelegt, daß bei eingebauter Prüfsonde der Gesamtmassenschwerpunkt nicht mit der Drehachse 19 zusammenfällt sondern so liegt, daß bei drehendem Rotierkopf auf die Sondenhebel ein der Kraft der Blattfedern entgegengerichtetes Drehmoment wirkt, das die Sondenhaltehebel in Richtung auf eine gegenstandsnähere Stellung drehen möchte. Die Massenverteilung und die Federkräfte der Blattfedern sind dabei so ausgelegt, daß die Sondenhaltehebel beim Hochdrehen des Rotierkopfes bis zu einer Drehzahl von beispielsweise ca. 1.500 Umdrehungen pro Minute eine gegenstandsferne Abhebestellung einnehmen, bei der der Bolzen 34 vom Anschlag 33 abgehoben ist. Bei Drehzahlen oberhalb dieser Grenzdrehzahl beginnt, durch zunehmende Fliehkräfte bewirkt, eine Zustellung der Prüfsonden in Richtung Prüfgegenstand, bis die Sondenhaltehebel bei ca. 3.500 Umdrehungen pro Minute ihre durch den Anschlag 33 vorgegebene gegenstandsnächste Prüfposition einnehmen, die sie auch bei höheren Drehzahlen unverändert beibehalten. Dieses System mit drehzahlabhängiger, selbsttätiger Abhebung der Prüfsonden hat unter anderem den Vorteil, daß bei nicht maßhaltigen oder gebogenen Anfängen oder Enden des durchlaufenden Prüfgutes das Prüfgut im Drehzahlbereich unterhalb der Grenzdrehzahl ohne Beschädigung der Prüfsonden in den Rotierkopf eingeführt werden kann. Ist das Prüfgut in den Rotierkopf eingetaucht, wird dieser wieder zur gewünschten Prüfdrehzahl hochgefahren. Läuft das Prüfgutende in den Rotierkopf ein, so wird dieser wieder bis auf Bereiche unterhalb der Grenzdrehzahl abgebremst, so daß sich die Sonden selbsttätig auf den größeren Durchmesser einstellen und somit nicht beschädigt werden können.

Das Verstellelement 25 hat auf seiner dem Trägerelement 21 zugewandten Rückseite eine umlaufende Ringnut, in die ein Dichtring 28 (Fig. 3) aus elastischem Kunststoffmaterial eingelegt ist. Dieser Dichtring wird zwischen Verstellelement 25 und Trägerelement 21 zusammengedrückt und schafft dadurch in diesem Bereich ausschließlich eine kraftschlüssige bzw. reibschlüssige Verbindung zwischen dem Verstellelement 25 und dem Rotor 15, mit dem das Trägerelement 21 drehfest verbunden ist. Hierdurch wird eine Selbsthemmung der Durchmesserverstellung geschaffen, welche eine spielfreie Durchmessereinstellung ermöglicht.

Ein wesentliches Merkmal erfindungsgemäßer Prüfeinrichtungen besteht nun darin, daß die beschriebenen Elemente der Durchmesserverstelleinrichtung zur Durchmesserverstellung gegeneinander bewegt werden können, ohne daß eine Öffnung des Gehäuses erforderlich ist. Hierzu hat die Verstelleinrichtung ein von außerhalb der Prüfeinrichtung zugängliches Betätigungsorgan 40, dessen Aufbau und Funktion insbesondere im Zusammenhang mit Fig. 3 beschrieben wird. Fig. 3 stellt in einer vertikalen Ansicht das horizontal von der Seite des Prüfeinrichtungsgehäuses 2 abstehende Betätigungsorgan 40 lagerichtig dar. Dagegen ist die Anordnung des Betätigungsorgans in Fig. 1 aus Darstellungsgründen um 90° um die Achse 5 versetzt gezeigt, während der tatsächliche Anbringungsort an der Gehäuseseite durch gestrichelte konzentrische Kreise 41 angedeutet ist.

An einer Seite des Prüfeinrichtungsgehäuses 2 ist ein horizontal abstehendes, zylindrisches Gehäuse 42 im Axialbereich des Rotorabschnitts 16 und des Verstellelementes 25 angebracht. In diesem Gehäuse ist das Betätigungsorgan 40 um seine als Organachse bezeichnete Längsachse 43 drehbar und entlang der Achse 43 radial zur Achse 5 verschiebbar gelagert. Das mittels eines griffgünstig gestalteten Handrades 44 manuell drehbare und verschiebbare Betätigungsorgan hat als wesentliches Element eine rotationssymmetrische Welle 45, die an ihrem der Prüfeinrichtung zugewandten Endabschnitt ein kegelstumpfförmiges Winkelzahnrad 46 und daran anschließend einen zylindrischen Fortsatz 47 aufweist. Das Handrad 44 ist an das aus dem Gehäuse 42 ragende Außenende der Welle angeschraubt. Innerhalb des Gehäuses hat die Welle einen an einem gesonderten Wellenteil ausgebildeten, radial abstehenden Ringbund 48 sowie eine konische Steuerschulter 49.

Das Betätigungsorgan ist unterhalb der Bruchlinie in einem Ruhezustand mit außenstehendem Handrad und oberhalb der Bruchlinie in einem eingerückten Zustand mit an der Außenseite des Gehäuses 42 aufliegendem, gestrichelt schraffiertem Handrad gezeigt. In dem ersten Verstellzustand nimmt das Winkelzahnrad 46 die mittels durchgezogenen Linien gezeigte Stellung außerhalb der drehbaren Elemente 16, 25 des Rotierkopfes an, während in dem strichpunktiert gezeigten inneren, dritten Verstellzustand das Winkelzahnrad in Eingriff mit den drehbaren Elementen 16, 25 des Rotierkopfes steht. Eine als zweiter Verstellzustand bezeichnete Zwischenstellung ist nicht dargestellt. Die drei dank dieser Konstruktion möglichen Verstellzustände der Verstelleinrichtung werden weiter unten im Detail beschrieben.

Der Verstelleinrichtung ist eine anhand von Figuren 3 und 4 näher erläuterte Sicherungseinrichtung 50 zugeordnet, die ausschließlich mit mechanisch zusammenwirkenden Elementen sicherstellt, daß eine Bewegung des Betätigungsorgans 40 in eine Verstellposition zum verstellwirksamen Eingriff an drehbaren Elementen des Prüfkopfes nur dann möglich ist, wenn der Rotierkopf steht. Die Sicherungseinrichtung 50 umfaßt eine achsparallel neben dem Betätigungsorgan 40 im Gehäuse 42 angebrachte innere Hülse 51, die auf der Seite des Handgriffs 44 aus dem Gehäuse 42 hinausragt und im Gehäuse parallel zur Achse 43 anschlagsbegrenzt verschiebbar ist, wobei der Anschlag durch ein an die Hülse 51 stirnseitig aufgeschraubtes, verbreitertes Kopfelement 52 gebildet wird. Am gegenüberliegenden, dem Rotor zugewandten Ende ist mittels eines Kugellagers 53 eine Sperrklinke 54 leichtgängig verschwenkbar angelenkt, wobei die Schwenkachse 55 der Sperrklinke parallel zur Rotordrehachse 5 verläuft. Ein rotornaher Kopfabschnitt 56 der Sperrklinke reicht bis in unmittelbare Nähe einer zylindrischen Umfangsfläche des Rotorabschnitts 16 nahe der Labyrinthdichtung 23. Im Inneren der Hülse 51 ist eine Spiraldruckfeder 57 angeordnet, die in den Figuren 3 und 4 oberhalb der Bruchlinie im ausgedehnten und unterhalb der Bruchlinie im zusammengedrückten Zustand gezeigt ist.

Die innere Hülse 51 wird von einer axial kürzeren äußeren Hülse 58 umschlossen, die relativ zu der inneren Hülse verschiebbar geführt ist. Die äußere Hülse 58 hat an ihrer äußeren Stirnseite eine Anschlagsfläche für den Ringbund 48 des Betätigungsorgans 40. Weiterhin ist ein diametraler Querstift 59 vorgesehen, der durch Langlöcher 60 der inneren Hülse hindurchgreift und der ein äußeres Widerlager für die Druckfeder 57 bildet, deren anderes Ende sich im rotornahen Bodenbereich der inneren Hülse 51 abstützt.

Eine vorzugsweise vorgesehene, weitere Sicherungseinrichtung umfaßt einen im Gehäuse 42 angebrachten elektrischen Schalter 65 mit einem mittels der Steuerschulter 49 des Betätigungsorgans 40 betätigbaren Schalterstift 66. Der Schalter 65 wirkt so mit dem elektrischen Antrieb 7 der Prüfeinrichtung zusammen, daß der Antrieb nicht gestartet werden kann, wenn sich der Schaltstift 66 in der bei eingerücktem Betätigungsorgan gegebenen, in Fig. 3 gezeigten Außenstellung befindet.

Im folgenden wird nun das Zusammenspiel der beschriebenen Elemente der Verstelleinrichtung näher erläutert. Dabei wird von einer Situation ausgegangen, bei der sich der Rotierkopf noch dreht und bei der sich das Betätigungsorgan in der in Fig. 3 unterhalb der Bruchlinie gezeigten Ruhestellung mit außen liegendem, durchgezogen schraffierten Handrad befindet. In diesem ersten Verstellzustand steht das Betätigungsorgan nicht in Eingriff mit dem Rotierkopf und es ist keine Prüfdurchmesserverstellung möglich, da sich das Winkelzahnrad 46 außer Eingriff mit dem Prüfkopf befindet. Das Betätigungsorgan ist mittels der Druckfeder 57, die über den Querstift 59, die äußere Hülse 58 und den Ringbund 48 auf das Betätigungsorgan wirkt, in diese Grundstellung vorgespannt.

Wird nun das Handrad Richtung Prüfkopf verschoben, so wird die Druckfeder 57 zusammengepreßt, was zunächst eine geringfügige Verschiebung der inneren Hülse 51 in Richtung Prüfkopf bewirkt. Dadurch kommt es zu einer Annäherung zwischen dem Kopfabschnitt 56 der Sperrklinke 54 und der Rotoraußenseite. Wenn sich der Rotor noch ausreichend schnell in Drehrichtung 64 dreht, so reicht bei Annäherung der Sperrklinke der erzeugte Luftzug aus, um die leichtgängig drehbare Sperrklinke in die in Fig. 4 strichpunktiert gezeigte Auslenkstellung zu verschwenken. In diesem Sperrzustand bildet die Sperrklinke einen Innenanschlag für die Bewegung der äußeren Hülse 58 und sperrt dadurch diese Axialbewegung. Damit ist auch ein weiteres Einrücken des Betätigungsorgans 40 auf ausschließlich mechanische Weise zuverlässig gesperrt. Diese Sperrwirkung der Sicherungseinrichtung 50 tritt bei ausreichend schnell rotierendem Rotierkopf ohne Berührungskontakt zwischen Sperrklinke und Rotor auf, so daß Reibungsverschließ dieser Elemente zuverlässig vermieden wird. Die Sicherungseinrichtung arbeitet auch bei sehr langsam drehendem Rotor. In diesem Fall kann die Sperrklinke bis zur Berührung zwischen Kopfabschnitt 56 und Rotorabschnitt 16 nach innen verschoben werden, bevor die Sperrklinke in die Sperrstellung ausklappt. Es tritt also höchstens ein sehr kurzer Berührungskontakt zwischen Sperrklinke und Rotor auf, der wegen der niedrigen Drehzahlen praktisch keinen Verschleiß verursacht.

Ein verstellwirksamer Eingriff zwischen Betätigungsorgan und Rotierkopf ist also nur bei stehendem Rotor möglich. Wenn der Rotor steht, so wird bei Veschiebung der äußeren Hülse Richtung Prüfkopf die Sperrklinke 54 nicht aus ihrer in Fig. 4 mit durchgezogenen Linien gezeigten Ruhestellung bewegt, die sich aufgrund der Drehlagerung der Sperrklinke außerhalb ihres Massenschwerpunktes schwerkraftbedingt selbsttätig einstellt. Befindet sich die Sperrklinke in ihrer Ruhestellung, so kann die äußere Hülse 58 bei Eindrücken des Betätigungsorgans 40 über die Sperrklinke geschoben und dadurch bis zum Anschlag an der prüfkopfnahen Gehäuseinnenseite unter Zusammendrücken der Druckfeder 57 verschoben.werden.

Da in der Regel nach Anhalten des Rotors die Radialbohrung 14 am Rotorrand nicht mit der Verschiebeachse 43 des Betätigungsorgans 40 zusammenfällt, ist zunächst ein Einschieben des Betätigungsorganes nur soweit möglich, bis die Stirnseite des Zylinderabschnitts 47 auf den zylindrischen Umfang des Rotorabschnitts 16 stößt. In diesem in Fig. 3 aus Gründen der Übersichtlichkeit nicht gezeichneten, zweiten Verstellzustand steht das Winkelzahnrad 46 schon in Teileingriff mit dem Zahnkranz 13 am Verstellelement 25, das mittels des Reibschlußkörpers 28 reibschlüssig mit dem Rotor gekoppelt ist. In diesem Verstellzustand ist eine Drehung des gesamten Prüfkopfes (Rotor und Verstellelement) durch Drehung des Handrades 44 möglich. Dabei kann so lange gedreht werden, bis die radiale Bohrung 14 in den Bereich der Betätigungsorganachse 43 gerät, so daß unter weiterer Einwärtsbewegung des Betätigungsorganes der Zylinderabschnitt 47 im wesentlichen spielfrei in die Bohrung 14 einführbar ist. In dieser Drehstellung des Prüfkopfes ist eine am Umfang von Verstellelement 25 und Rotorabschnitt 16 vorgesehene Skala zur Kontrolle der Durchmessereinstellung durch ein geeignet angeordnetes Fenster im Gehäuse der Prüfeinrichtung sichtbar. Durch den Eingriff des Betätigungsorganes in den Rotor ist dieser nun formschlüssig gegen Verdrehung gesichert. Außerdem ist der Schalterstift 66 des Schalters 65 ausgerückt, so daß der elektrische Antrieb stromlos ist und nicht eingeschaltet werden kann.

In diesem dritten Verstellzustand kann nun eine Prüfdurchmesserverstellung durch Drehung des Betätigungsorganes durchgeführt werden. Dabei dient die Bohrung 14 als Widerlager für die Welle 45 bzw. das Winkelzahnrad 46, das jetzt in vollem Eingriff mit dem Zahnkranz 13 steht. Durch Drehung des Betätigungsorganes wird die durch den Dichtring 28 aufgebrachte Haftreibung und danach auch die in diesem Bereich auftretende Gleitreibung überwunden, so daß das Verstellelement 25 gegenüber dem (unbewegten) Rotor verdreht wird. Diese Relativverdrehung bewirkt auf die schon eingangs der Beschreibung beschriebene Weise über die Spiralnuten 27 und die hebelartigen Verstellglieder 30 eine Verstellung des Innenanschlages 33 für die Sondenhalterhebel 18 und damit eine Verstellung des Prüfdurchmessers.

Ist die dank der Reibschlußverbindung (Reibschlußelement 28) spielfreie Durchmesserverstellung abgeschlossen, so wird das Betätigungsorgan losgelassen, wodurch es mittels der Druckfeder 57 selbsttätig in seine Ruhestellung zurückkehrt.

## Patentansprüche

1. Prüfeinrichtung zum Prüfen von langgestreckten Gegenständen, insbesondere Drähten, Stangen, Rohren o. dgl., mit einem zum Hindurchführen der Gegenstände ausgebildeten, um eine Rotierkopfachse drehbaren Rotierkopf, an dem mindestens eine an beweglichen Sondenhaltemitteln angeordnete, auf im wesentlichen kreisförmigen Umlaufbahnen um den Gegenstand führbare Prüfsonde zur zerstörungsfreien Prüfung von oberflächennahen Bereichen des Gegenstandes vorgesehen ist, sowie mit einer Verstelleinrichtung zur Verstellung des Durchmessers der Umlaufbahn, wobei die Verstelleinrichtung mindestens ein von außerhalb der Prüfeinrichtung zugängliches Betätigungsorgan (40) zur Betätigung der Verstelleinrichtung aufweist, **dadurch gekennzeichnet, dass** das Betätigungsorgan (40) beweglich gelagert und derart mit verstellwirksam beweglichen Elementen (16, 25) der Verstelleinrichtung mechanisch koppelbar ist, dass die Verstelleinrichtung durch Bewegung des Betätigungsorgans (40) zwischen mehreren Verstellzuständen umschaltbar ist, wobei in einem ersten Verstellzustand das Betätigungsorgan (40) nicht in Eingriff mit dem Rotierkopf steht, so dass kein Verstelleingriff mit dem Rotierkopf vorliegt, in einem zweiten Verstellzustand durch Bewegung des Betätigungsorgans (40) eine kontrollierte Rotation des Rotierkopfs durchführbar ist und in einem dritten Verstellzustand eine Durchmesserverstellung des Prüfdurchmessers durchführbar ist.

2. Prüfeinrichtung nach Anspruch 1 **dadurch gekennzeichnet, daß** die Verstelleinrichtung ohne Öffnung eines Gehäuses (2) der Prüfeinrichtung betätigbar ist.

3. Prüfeinrichtung nach Anspruch 1 oder 2, **dadurch gekennzeichnet, daß** ihr eine automatische Manipulationseinrichtung zur vestellwirksamen Betätigung des Betätigungsorgans (40) zugeordnet oder zuordenbar ist.

4. Prüfeinrichtung nach einem der vorhergehenden Ansprüche, **dadurch gekennzeichnet, daß** die Verstelleinrichtung als vollmechanische Verstelleinrichtung ausgebildet ist.

5. Prüfeinrichtung, nach einem der vorhergehenden Ansprüche, **dadurch gekennzeichnet, daß** zur Betätigung der Verstelleinrichtung ein einziges Betätigungsorgan (40) vorgesehen ist.

6. Prüfeinrichtung nach einem der vorhergehenden Ansprüche, **dadurch gekennzeichnet, daß** das Betätigungsorgan (40) um eine Organachse (43) drehbar und, unabhängig von der Drehung, parallel zur Organachse verschiebbar gelagert ist.

7. Prüfeinrichtung nach einem der vorhergehenden Ansprüche, **dadurch gekennzeichnet, daß** der Verstelleinrichtung eine Sicherungseinrichtung (50) zugeordnet ist, die derart ausgebildet ist, daß eine Bewegung des Betätigungsorgans (40) in einen Verstellzustand mit verstellwirksamem Eingriff am Rotierkopf nur bei ruhendem Rotierkopf durchführbar ist.

8. Prüfeinrichtung nach Anspruch 7, **dadurch gekennzeichnet, dass** die Sicherungseinrichtung (50) vollmechanisch arbeitet.

9. Prüfeinrichtung nach Anspruch 7 oder 8, **dadurch gekennzeichnet, daß** die Sicherungseinrichtung (50) mindestens ein in Wirkverbindung mit dem Rotierkopf bewegbares Sperrorgan (54) aufweist, das durch Drehung des Rotierkopfs in eine die Bewegung des Betätigungsorgans (40) sperrende Sperrstellung bewegbar, insbesondere verschwenkbar ist, wobei vorzugsweise das Sperrorgan (54) zumindest bei hohen Drehgeschwindigkeiten des Rotierkopfes berührungslos mit dem Rotierkopf zusammenwirkt.

10. Prüfeinrichtung nach einem der vorhergehenden Ansprüche, **dadurch gekennzeichnet, daß** die Verstelleinrichtung spielfrei einstellbar ist.

11. Prüfeinrichtung nach einem der vorhergehenden Ansprüche, **dadurch gekennzeichnet, daß** der Rotierkopf einen um eine Prüfkopfachse (5) drehbaren Rotor (15,16,21) zum Tragen der Sondenhaltemittel und mindestens ein koaxial mit dem Rotor angeordnetes, relativ zum Rotor verdrehbares, vorzugsweise nach Art einer Kurvenscheibe ausgebildetes Verstellelement (25) aufweist, das derart mit den Sondenhaltemitteln (18) gekoppelt ist, daß die Sondenhaltemittel durch Relativverdrehung zwischen Rotor und Verstellelement zur Änderung des Prüfdurchmessers verstellbar sind.

12. Prüfeinrichtung nach Anspruch 11, **dadurch gekennzeichnet, daß** der Rotor (15,16,21) und das Verstellelement (25) mittels einer Reibschlussverbindung zur Erzeugung einer Selbsthemmung der Verstelleinrichtung reibschlüssig miteinander verbunden sind, wobei die Reibschlussverbindung vorzugsweise derart ausgebildet ist, daß eine Reibungskraft erzeugbar ist, die mit steigender Drehzahl des Rotierkopfes zunimmt.

13. Prüfeinrichtung nach Anspruch 11 oder 12, **dadurch gekennzeichnet, daß** zwischen dem Rotor (15,16,21) und dem Verstellelement (25) mindestens ein gesondertes, elastisches Reibschlußelement (28) zur Bildung einer Reibschlußverbindung zwischen Rotor und Verstellelement vorgesehen ist, wobei das Reibschlusselement vorzugsweise nach Art eines Dichtrings aus elastischem Material ausgebildet ist.

14. Prüfeinrichtung nach einem der vorhergehenden Ansprüche, **dadurch gekennzeichnet, daß** die Verstelleinrichtung im wesentlichen vollständig verschmutzungsgeschützt abgedichtet ist.

15. Prüfeinrichtung nach einem der vorhergehenden Ansprüche, **dadurch gekennzeichnet, daß** sie ein den Rotierkopf umgebendes Gehäuse (2) hat, das einen die Prüfsonden (19) umschließenden Prüfbereich (11) und einen bewegbare Elemente (16,25) der Verstelleinrichtung umschließenden Gehäuseinnenraum aufweist und daß der Prüfsondenbereich (11) zum Gehäuseinnenraum mittels Abdichtmitteln (23) verschmutzungsdicht abgedichtet ist.

16. Prüfeinrichtung nach Anspruch 15, **dadurch gekennzeichnet, daß** die Abdichtmittel am Umfang des Rotierkopfes zwischen Rotierkopf und Gehäuse (2) eine mit berührungsfrei zusammenarbeitenden Dichtelementen arbeitende Verschmutzungsdichtung, insbesondere nach Art einer Labyrinthdichtung (23), aufweisen.

17. Prüfeinrichtung nach einem der vorhergehenden Ansprüche, **dadurch gekennzeichnet, daß** die Sondenhaltemittel (18) an einer Vorderseite eines vorzugsweise scheibenförmigen Rotorabschnitts (16) und ein gegenüber dem Rotorabschnitt verdrehbares Verstellelement (25) gegenüber einer Rückseite des Rotorabschnitts angeordnet ist und daß zur Kopplung zwischen Verstellelement und Sondenhaltemittel ein den Rotorabschnitt (16) axial durchsetzendes, vorzugsweise schwenkbares Verstellglied (30) vorgesehen ist, wobei vorzugsweise der Bereich der Durchführung des Verstellgliedes verschmutzungsdicht abgedichtet ist.

18. Prüfeinrichtung nach einem der vorhergehenden Ansprüche, **dadurch gekennzeichnet, daß** ein elektrischer Antrieb (7) zum Antreiben des Rotierkopfs vorgesehen ist und daß eine elektrische Versorgung des Antriebes durch Bewegung des Betätigungsorgans (40) in einen Verstellzustand mit verstellwirksamem Eingriff zum Rotierkopf unterbrechbar ist, insbesondere mittels eines durch das Betätigungsorgans (40) betätigbaren elektrischen Schalters (65).

19. Prüfeinrichtung nach einem der vorhergehenden Ansprüche, **dadurch gekennzeichnet, daß** die Sondenhaltemittel (18) als um eine Schwenkachse (19) schwenkbare Sondenhaltehebel ausgebildet sind, vorzugsweise mit einem die Prüfsonde (20) tragenden ersten Arm und einem vorzugsweise ein Gegengewicht bildenden oder tragenden zweiten Arm.

20. Prüfeinrichtung nach einem der vorhergehenden Ansprüche, **dadurch gekennzeichnet, daß** die Sondenhaltemittel, insbesondere die Sondenhaltehebel (18) abhängig von der Drehzahl des Rotierkopfes selbsttätig zwischen einer gegenstandsnahen Stellung und einer Abhebestellung derart verstellbar sind, daß unterhalb einer Grenzdrehzahl die Abhebestellung eingenommen wird.

21. Prüfeinrichtung nach Anspruch 19 oder 20, **dadurch gekennzeichnet, daß** ein Sondenhaltehebel (18), insbesondere mittels einer Blattfeder (35) in eine Abhebestellung vorgespannt ist und daß er derart außerhalb seines Massenschwerpunktes drehbar gelagert ist, daß fliehkraftabhängig ein in Richtung gegenstandsnaher Stellung wirkendes Drehmoment erzeugbar ist.

22. Prüfeinrichtung nach einem der vorhergehenden Ansprüche, **dadurch gekennzeichnet, daß** die Verstelleinrichtung zur Prüfdurchmesserverstellung eines Sondenhaltemittels ein dem Sondenhaltemittel zugeordnetes Verstellglied (30) aufweist, das als verstellbarer Anschlag zur Festlegung der gegenstandsnahen Stellung des Sondenhaltemittels ausgebildet ist, wobei vorzugsweise das Verstellglied nahe der Prüfsonde (19) am Sondenhaltemittel (18) angreift.

## Claims

1. Test device for testing elongated objects, particularly wires, bars, pipes, etc., with a rotary head rotatable about a rotary head axis constructed for the passage of objects on which is provided a test probe placed on movable probe holding means and guidable on substantially circular orbits around the object for the nondestructive testing of surface-near areas of the object, as well as with an adjustment device for adjusting the diameter of the orbit, the adjustment device having at least one actuator (40) accessible from outside the test device for actuating the adjustment device, **characterized in that** the actuator (40) is movably mounted and can be mechanically coupled to adjustment-effectively movable elements (16, 25) of the adjustment device in such a way that said adjustment device can be switched between several adjustment states by moving the actuator (40), where in a first adjustment state the actuator (40) is not engaged with the rotary head, so that there is no adjustment engagement with the rotary head, in a second adjustment state a controlled rotary head rotation can take place by moving the actuator (40) and in a third adjustment state a test diameter adjustment can take place.

2. Test device according to claim 1, **characterized in that** the adjustment device can be operated without opening the test device casing (2).

3. Test device according to claim 1 or 2, **characterized in that** with it is or can be associated an automatic manipulator for the adjustment-effective actuation of actuator (40).

4. Test device according to one of the preceding claims, **characterized in that** the adjustment device is constructed as a completely mechanical adjustment device.

5. Test device according to one of the preceding claims, **characterized in that** a single actuator (40) is provided for actuating the adjustment device.

6. Test device according to one of the preceding claims, **characterized in that** actuator (40) is rotatable about an actuator axis (43) and is displaceably mounted parallel to the actuator axis, independently of the rotation.

7. Test device according to one of the preceding claims, **characterized in that** with the adjustment device is associated a safety device (50) constructed in such a way that a movement of actuator (40) into an adjustment state with adjustment-effective engagement on the rotary head is only possible with said rotary head stationary.

8. Test device according to claim 7, **characterized in that** the safety device (50) operates completely mechanically.

9. Test device according to claim 7 or 8, **characterized in that** the safety device (50) has at least one locking member (54) movable in operative connection with the rotary head and which can be moved by rotary head rotation into a locking position blocking the movement of actuator (40), particularly by pivoting and preferably the locking member (54) cooperates in non-contacting manner with the rotary head, at least at high rotary head rotation speeds.

10. Test device according to one of the preceding claims, **characterized in that** the adjustment device can be set in play-free manner.

11. Test device according to one of the preceding claims, **characterized in that** the rotary head has a rotor (15, 16, 21) rotatable about a test head axis (5) for carrying the probe holding means and at least one adjustment element (25) positioned coaxially to the rotor, rotatable relative to the rotor and preferably constructed in the manner of a disk cam, which is coupled to the probe holding means (18) in such a way that said means can be adjusted by relative rotation between rotor and adjustment element for modifying the test diameter.

12. Test device according to claim 11, **characterized in that** the rotor (15, 16, 21) and adjustment element (25) can be interconnected by frictional engagement by means of a frictional engagement connection for bringing about a self-locking of the adjustment device, the frictional engagement connection preferably being constructed in such a way that a frictional force can be produced which increases with rising rotary head speed.

13. Test device according to claim 11 or 12, **characterized in that** between rotor (15, 16, 21) and adjustment element (25) there is at least one separate, elastic frictional engagement element (28) provided for creation of a frictional engagement connection between rotor and adjustment element, said frictional engagement element preferably being constructed in the manner of an elastic material ring gasket.

14. Test device according to one of the preceding claims, **characterized in that** the adjustment device is sealed in a substantially completely dirt-protected manner.

15. Test device according to one of the preceding claims, **characterized in that** it has a casing (2) surrounding the rotary head which is provided with a test area (11) surrounding the test probes (19) and a casing interior enveloping movable elements (16, 25) of the adjustment device and that the test probe area (11) is sealed in dirt-tight manner with respect to the casing interior by sealing means (23).

16. Test device according to claim 15, **characterized in that** on the circumference of the rotary head between the latter and the casing (2), the sealing means have a dirt seal, particularly in the manner of a labyrinth packing (23) operating with contact-free cooperating sealing elements.

17. Test device according to one of the preceding claims, **characterized in that** the probe holding means (18) are located on a front side of a preferably disk-shaped rotor portion (16) and an adjustment element (25) rotatable with respect to the rotor portion with respect to a rear side of the rotor portion and that for coupling between the adjustment element and the probe holding means there is a preferably pivotable adjustment member (30) axially traversing the rotor portion (16) and preferably the adjustment member passage area is sealed in dirt-tight manner.

18. Test device according to one of the preceding claims, **characterized in that** an electric drive (7) is provided for driving the rotary head and that an electric power supply of the drive can be interrupted by moving the actuator (40) into an adjustment state with adjustment-effective engagement with the rotary head, particularly by means of an electric switch (63) actuatable by actuator (40).

19. Test device according to one of the preceding claims, **characterized in that** the probe holding means (18) are constructed as probe holding levers pivotable about a pivoting axis (19), preferably with a first arm carrying the test probe (20) and a second arm preferably carrying or forming a counterweight.

20. Test device according to one of the preceding claims, **characterized in that** the probe holding means, particularly the probe holding levers (18), are adjustable independently of the rotary head speed in automatic manner between an object-near position and a lifting position in such a way that the lifting position is assumed below a limit speed.

21. Test device according to claim 19 or 20, **characterized in that** a probe holding means (18) is pretensioned into a lifting position, particularly by means of a leaf spring (35), and that it is rotatably mounted outside its mass centre in such a way that in centrifugal force-dependent manner a torque acting towards the object-near position can be produced.

22. Test device according to one of the preceding claims, **characterized in that** for the test diameter adjustment of a probe holding means, the adjustment device has an adjustment member (30) associated with the probe holding means and which is constructed as an adjustable stop for fixing the object-near position of the probe holding means and preferably the adjustment member acts on the probe holding means (18) close to the test probe (19).

## Revendications

1. Dispositif de contrôle pour le contrôle d'objets allongés, notamment des fils, des tiges, des tuyaux ou similaires, présentant une tête rotative réalisée pour être traversée par des objets et tournant autour d'un axe de tête rotative, sur laquelle on prévoit au moins une sonde de contrôle disposée sur des dispositifs de support de sonde movibles, pouvant être guidée sur des orbites essentiellement circulaires autour de l'objet et prévue pour le contrôle non destructif de zones proches de la surface de l'objet, ainsi qu'un dispositif d'ajustage pour l'ajustage du diamètre d'orbite, sachant que le dispositif d'ajustage présente au moins un organe d'actionnement (40) accessible de l'extérieur du dispositif de contrôle et prévu pour l'actionnement du dispositif d'ajustage, **caractérisé en ce que** l'organe d'actionnement (40) est logé de manière mobile et peut être couplé mécaniquement avec des éléments (16, 25) mobiles du dispositif d'ajustage, dont le mouvement entraîne un effet d'ajustage de manière que par le mouvement de l'organe d'actionnement (40) le dispositif d'ajustage peut être commuté entre plusieurs états d'ajustage, sachant que dans un premier état d'ajustage l'organe d'actionnement (40) ne s'engage pas sur la tête rotative et qu'il n'y a donc aucun engagement d'ajustage sur la tête rotative, et que dans un deuxième état d'ajustage on peut produire une rotation contrôlée de la tête rotative en mouvant l'organe d'actionnement (40) et que dans un troisième état d'ajustage on peut effectuer un ajustage du diamètre de contrôle.

2. Dispositif de contrôle d'après la revendication 1, **caractérisé en ce que** le dispositif d'ajustage peut être actionné sans ouvrir le boîtier (2) du dispositif de contrôle.

3. Dispositif de contrôle d'après la revendication 1 ou 2, **caractérisé en ce qu'**un dispositif de manipulation automatique pour effectuer un actionnement dont le mouvement entraîne un effet d'ajustage lui est assigné ou peut lui être assigné.

4. Dispositif de contrôle d'après une des revendications précédentes, **caractérisé en ce que** le dispositif d'ajustage est réalisé comme dispositif d'ajustage entièrement mécanique.

5. Dispositif de contrôle d'après une des revendications précédentes, **caractérisé en ce qu'**on prévoit un seul organe d'actionnement (40) pour l'actionnement du dispositif d'ajustage.

6. Dispositif de contrôle d'après une des revendications précédentes, **caractérisé en ce que** l'organe d'actionnement (40) est rotatif autour d'un axe d'organe (43) et qu'il est logé de manière à être déplaçable parallèlement par rapport à l'axe d'organe indépendamment de la rotation.

7. Dispositif de contrôle d'après une des revendications précédentes, **caractérisé en ce qu'**au dispositif d'ajustage est associé un dispositif de sécurité (50) réalisé de manière qu'un mouvement de l'organe d'actionnement (40) vers un état d'ajustage dans lequel l'engagement avec la tête rotative entraîne un effet d'ajustage ne puisse être effectué que si la tête rotative est immobile.

8. Dispositif de contrôle d'après la revendication 7, **caractérisé en ce que** le dispositif de sécurité (50) opère de manière entièrement mécanique.

9. Dispositif de contrôle d'après la revendication 7 ou 8, **caractérisé en ce que** le dispositif de sécurité (50) présente au moins un organe de verrouillage (54) qui est movible par un lien opératoire (*Wirkverbindung*) avec la tête rotative et qui peut être déplacé et notamment pivoté par une rotation de la tête rotative dans une position verrouillée, dans laquelle le mouvement de l'organe d'actionnement (40) est bloqué, sachant que de préférence l'organe de verrouillage (54) agit sans contact avec la tête rotative au moins quand la tête de rotation tourne à grande vitesse.

10. Dispositif de contrôle d'après une des revendications précédentes, **caractérisé en ce que** le dispositif d'ajustage peut être ajusté sans jeu.

11. Dispositif de contrôle d'après une des revendications précédentes, **caractérisé en ce que** la tête rotative présente un rotor (15, 16, 21) tournant autour d'un axe de tête de contrôle (5) pour porter les dispositifs de support de sonde, et au moins un élément d'ajustage (25) coaxial par rapport au rotor, rotatif par rapport au rotor et réalisé de préférence à la manière d'un disque-came, qui est couplé avec les dispositifs de support de sonde (18) de manière que les dispositifs de support de sonde soient ajustables par une rotation relative entre le rotor et l'élément d'ajustage pour une modification du diamètre de contrôle.

12. Dispositif de contrôle d'après la revendication 11, **caractérisé en ce que** le rotor (15, 16, 21) et l'élément d'ajustage (25) sont raccordés entre eux à engagement par friction au moyen d'un raccordement par engagement à friction pour produire un blocage automatique, sachant que le raccordement par engagement à friction est réalisé de préférence de manière qu'on puisse produire une force de frottement qui augmente quand la vitesse de rotation augmente.

13. Dispositif de contrôle d'après la revendication 11 ou 12, **caractérisé en ce qu'**entre le rotor (15, 16, 21) et l'élément d'ajustage (25) on prévoit au moins un élément à engagement par friction (28) distinct et élastique pour constituer un raccordement par engagement à friction entre le rotor et l'élément d'ajustage, sachant que l'élément à engagement par friction est réalisé de préférence à la manière d'un joint d'étanchéité en un matériau élastique.

14. Dispositif de contrôle d'après une des revendications précédentes, **caractérisé en ce que** le dispositif de contrôle est étanché en étant en substance complètement à l'abri des salissures.

15. Dispositif de contrôle d'après une des revendications précédentes, **caractérisé en ce qu'**il présente un boîtier (2) qui entoure la tête rotative et qui présente une zone de contrôle (11) enfermant les sondes de contrôle (19) et un espace intérieur de boîtier enfermant les éléments mobiles (16, 25) du dispositif d'ajustage et **en ce que** la zone des sondes de contrôle (11) est étanchée vers l'espace intérieur du boîtier en étant étanche aux salissures par des moyens d'étanchement (23).

16. Dispositif de contrôle d'après la revendication 15, **caractérisé en ce que** les moyens d'étanchement présentent au périmètre de la tête rotative, entre la tête rotative et le boîtier (2), un joint étanche aux salissures opérant avec des éléments d'étanchéité qui travaillent sans contact entre eux, notamment à la manière d'un joint à labyrinthe (23).

17. Dispositif de contrôle d'après une des revendications précédentes, **caractérisé en ce que** les dispositifs de support de sonde (18) sont disposés à une face antérieure d'une section de rotor (16) préférablement en forme de disque et qu'un élément d'ajustage (25) rotatif par rapport à la section de rotor est disposé vis-à-vis d'une face arrière de la section de rotor et **en ce que** pour le couplage entre l'élément d'ajustage et des dispositifs de support de sonde on prévoit une pièce d'ajustage (30) préférablement pivotable et traversant axialement la section de rotor (16), sachant que de préférence la zone de traversement de la pièce d'ajustage est rendue étanche aux salissures.

18. Dispositif de contrôle d'après une des revendications précédentes, **caractérisé en ce qu'**on prévoit un moteur électrique (7) pour l'entraînement de la tête rotative et **en ce qu'**une alimentation en courant électrique du moteur électrique peut être interrompue par un mouvement de l'organe d'actionnement (40) dans un état d'ajustage qui produit un engagement avec un effet d'ajustage sur la tête rotative, notamment au moyen d'un commutateur électrique (65) qui peut être actionné par l'organe d'actionnement (40).

19. Dispositif de contrôle d'après une des revendications précédentes, **caractérisé en ce que** les dispositifs de support de sonde (18) sont réalisés comme leviers de support de sonde pivotables autour d'un axe de pivotement (19), de préférence avec un premier bras portant la sonde de contrôle (20) et un deuxième bras constituant ou portant de préférence un contrepoids.

20. Dispositif de contrôle d'après une des revendications précédentes, **caractérisé en ce que** les dispositifs de support de sonde, notamment les leviers de support de sonde (18) sont réglables automatiquement entre une position à proximité de l'objet et une position de soulèvement en fonction de la vitesse de rotation de la tête rotative, sachant que la position de soulèvement est prévue pour des vitesses de rotation au-dessous d'une certaine vitesse limite de rotation.

21. Dispositif de contrôle d'après la revendication 19 ou 20, **caractérisé en ce qu'**un levier de support de sonde (18) est précontraint dans la position de soulèvement, notamment au moyen d'un ressort à lame (35) et **en ce qu'**il est logé de manière rotative en dehors de la positon de son centre de gravité, de manière qu'on puisse produire, en fonction de la force centrifuge, un couple qui pousse vers une position proche de l'objet.

22. Dispositif de contrôle d'après une des revendications précédentes, **caractérisé en ce que** le dispositif d'ajustage présente pour un ajustage du diamètre de contrôle d'un dispositif de support de sonde une pièce d'ajustage (30), qui est associée au dispositif de support de sonde et qui est réalisée comme arrêt pour fixer le dispositif de support de sonde dans la position proche de l'objet, sachant que de préférence la pièce d'ajustage s'engage sur le dispositif de support de sonde (18) à proximité de la sonde de contrôle (19).
